# EUROPEAN PATENT APPLICATION

(11) **EP 4 465 012 A1**
(43) Date of publication of application: **20.11.2024**
(21) Application number: 22920229.6
(22) Date of filing: 13.01.2022
(51) Int. Cl.: G01N 1/10, G01N 33/48

(54) **COLLECTION METHOD, TEST METHOD, CONTAINER, CENTRIFUGE AND TEST SYSTEM**

(71) Applicant: HITACHI HIGH-TECH CORPORATION, Tokyo 105-6409 (JP)
(72) Inventor: SUGIYAMA Kiyotaka, Tokyo 105-6409 (JP); FUJITA Hiroko, Tokyo 105-6409 (JP); SONEHARA Tsuyoshi, Tokyo 105-6409 (JP)
(74) Representative: MERH-IP Matias Erny Reichl Hoffmann Patentanwälte PartG mbB
(86) International application number: PCT/JP2022/000884
(87) International publication number: WO 2023/135704

(57) **Abstract**

A simple and stable method by which samples in different forms can be acquired at once from a blood sample. A method for collecting microorganisms that comprises: introducing a blood sample 200 into a first storage part 100 of a container 1, said container 1 being provided with the first storage part 100 having a first flow path 103 and a second flow path 104 of different heights on the side face, a second storage part 101 and a third storage part 102; centrifuging the blood sample 200 at a first centrifugal force to separate into a bacterial solution 202 containing microorganisms and a solution 203 containing blood cells; centrifuging the blood sample 200 at a second centrifugal force greater than the first centrifugal force and moving the solution 203 through the first flow path 103 to the second storage part 101; centrifuging the second solution 203 at a third centrifugal force to separate into a bacterial solution 206 containing microorganisms and blood components 207 containing blood cells; and centrifuging the solution 203 at a fourth centrifugal force greater than the third centrifugal force and moving the bacterial solution 206 through the second flow path 104 to the third storage part 102.

## Description

### Technical Field

The present invention relates to a collection method, a test method, a container, a centrifuge, and a test system. More specifically, the invention relates to a collection method for microorganisms that separates a sample containing impurities such as blood cells into a solution containing microorganisms such as bacteria and a solution containing blood cells, a test method for microorganisms, a container, a centrifuge, and a test system.

### Background Art

Sepsis is an infectious disease with a high mortality rate, and it is important to provide appropriate treatment promptly. Normally, sepsis is determined by a blood culture test to determine whether bacteria are present in blood which is a sterile sample. In general, a smear test is performed thereafter, a positive blood culture sample is separated and cultured, and an identification test for identifying a type of bacteria is performed on obtained colonies, and a sensitivity test for measuring the sensitivity of the bacteria to antibacterial agents is performed. In the series of tests described above, one day is required for a blood culture test, one day is required for a separation culture, and one day is required for the sensitivity test, and thus a test time of two to three days is required as a whole. Therefore, it currently takes two to three days to determine whether treatment with appropriate antibacterial agents is performed, and when ineffective antibacterial agents are administered, the mortality rate is extremely high.

Currently, attempts to speed up the series of infectious disease tests are being actively researched. For example, colorimetric and fluorescent methods used in the related art are golden standards for the bacterial identification test. Currently, a method for performing the identification test using mass spectrometry according to matrix assisted laser desorption/ionization-time of flight (MALDI-TOF) is developed, and the method is remarkably spread from the viewpoint of speeding up of the test and a cost merit. Although turbidimetry is used in relation to the sensitivity test in the related art, a method for detecting bacteria with high sensitivity using a laser and a method for predicting the sensitivity based on growth or a morphological change of bacteria using a microscope are developed. In this way, the test is being speeded up. Meanwhile, the separation culture still remains as one time-consuming test step, which is a bottleneck in shortening a test time. Here, if it is possible to remove non-bacterial substances such as blood cell components and other impurities from the positive blood culture sample and extract only the bacteria, the separation culture step can be omitted, leading to shortening the test time.

Since the identification test and the sensitivity test are performed as a set in a normal bacteria test, when the bacteria are extracted from the positive blood culture sample, it is necessary to prepare a sample suitable for both tests. If a sample suitable for both tests can be obtained by a single operation, the test time can be shortened and a labor of the test can be saved, which will bring a great merit to a user. For example, in the case of identification test, it is considered to use mass spectrometry according to MALDI-TOF, which is widely used. In MALDI, a dried sample is irradiated with a laser to perform desorption and ionization of the sample. Therefore, it is preferable that an amount of water in the sample is small, and from the viewpoint of detection sensitivity, an amount of bacteria of 10⁵ colon forming units (CFU) or more is required.

Meanwhile, in the case of the sensitivity test, a liquid containing living bacteria is used as a sample in turbidimetry or a rapid method utilizing a microscope or the like which is currently developed. In this case, for example, a bacterial solution whose concentration is adjusted within a range of 10⁵ to 10⁶ CFU/mL is used. When preparing the bacterial solution, a method called McFarland turbidimetry is generally used, and in order to accurately adjust a bacterial concentration, it is necessary to prepare several hundred µL of a 10⁸ CFU/mL solution. Therefore, an amount of bacteria required is greater than in the case of the identification test. Therefore, in order to rapidly perform a series of bacteria tests, it is necessary to extract bacteria from a relatively large volume of the positive blood culture sample of several mL or more, and to obtain a plurality of samples in different solid and liquid forms and different amounts of bacteria at a time.

PTL 1 discloses a method for separating bacteria from a blood sample and extracting protein of the bacteria. PTL 2 discloses a method for decomposing blood cells with a protease, performing an expansion treatment with a low-tension solution, and selectively destroying only blood cell components using a surfactant. PTL 3 discloses a method for separating a specific component from a blood sample.

### Citation List

### Patent Literature

PTL 1: JP2012-532618A
PTL 2: WO2019/097752
PTL 3: JP2006-242872A

### Summary of Invention

### Technical Problem

According to the method in PTL 1, a sample suitable for identification by mass spectrometry using MALDI can be obtained. However, since the treatment is performed with a reagent that destroys a bacterial cell wall, the sample prepared by the method does not contain living bacteria, and cannot be used for a sensitivity test.

According to the method in PTL 2, it is possible to sufficiently destroy and remove the blood cell component. The treatment includes several washing steps such as repeated centrifugation and solution replacement, making the treatment complicated and time-consuming. Although it is easy to obtain a liquid sample, in order to obtain a solid sample, additional operations such as centrifugation and removal of the supernatant are essential, and thus the number of operation steps increases. In order to obtain samples with different amounts of bacteria, it is necessary for a user to control a dispensing amount and the like of a bacterial solution, and accuracy thereof depends on a technique of dispensing and the like, and the operation is also complicated.

In the method in PTL 3, it is possible to easily separate the specific component from the blood sample in one step by rotating the blood sample like a spinning top. However, it is difficult to obtain samples in different solid and liquid forms for use in a series of bacteria tests. Since a filter such as a porous material is used for separation of the blood sample, it is not suitable for a use of rapidly treating a sample of several mL to 10 mL in relation to a problem of clogging.

The methods disclosed in PTL 1 and PTL 2 disclose a method for extracting bacteria from a blood sample, whereas in either method, it is not possible to obtain a solid bacterial sample required for the identification test and a liquid highly concentrated bacterial sample required for the sensitivity test at a time. In the method disclosed in PTL 3, a blood sample can be pretreated in a simple manner, whereas there is a problem in obtaining samples with different solid and liquid forms from large volume of samples at a time.

The invention has been made in view of such a situation, and an object of the invention is to provide a collection method, a test method, a container, a centrifuge, and a test system for obtaining samples in different forms from samples at a time by a simple and stable method.

### Solution to Problem

In order to solve the above problems, a collection method according to the invention includes: introducing a sample into a first storage part of a container including the first storage part having a first flow path and a second flow path of different heights on a side surface, a second storage part connected to the first storage part via the first flow path, and a third storage part connected to the first storage part via the second flow path; centrifuging the sample introduced into the first storage part with a first centrifugal force to separate the sample into a first solution containing a first component and a second solution containing a second component; centrifuging the sample stored in the first storage part with a second centrifugal force greater than the first centrifugal force and moving the first solution to the second storage part via the first flow path; centrifuging the second solution stored in the first storage part with a third centrifugal force to separate the second solution into a third solution containing the first component and a fourth solution containing the second component; and centrifuging the second solution stored in the first storage part with a fourth centrifugal force greater than the third centrifugal force and moving the third solution to the third storage part via the second flow path.

### Advantageous Effects of Invention

According to the invention, samples in different forms can be obtained at a time from samples by a simple and stable method.

Additional features related to the invention will be clarified based on the description of the present description and the accompanying drawings. Problems, configurations, and effects other than those described above will become apparent in the following description of embodiments. The description of the invention is merely a typical example, and does not limit the scope of the claims or application examples in any sense.

### Brief Description of Drawings

[FIG. 1] FIG. 1 is a cross-sectional view of a container according to Embodiment 1.
[FIG. 2] FIG. 2 is a transition diagram of a blood sample in the container, showing a method for obtaining samples in different forms from the blood sample introduced into the container according to Embodiment 1.
[FIG. 3] FIG. 3 is a diagram showing a relationship between a centrifugal acceleration and a time in the method for obtaining the samples in different forms from the blood sample.
[FIG. 4] FIG. 4 is a cross-sectional view showing a height of a flow path formed in the container according to Embodiment 1.
[FIG. 5] FIG. 5 is a block diagram showing a configuration of a test system according to Embodiment 1.
[FIG. 6] FIG. 6 is a block diagram showing a configuration of a centrifuge according to Embodiment 1.
[FIG. 7] FIG. 7 is a flowchart showing a method for obtaining the samples in different forms from the blood sample and a test using the sample.

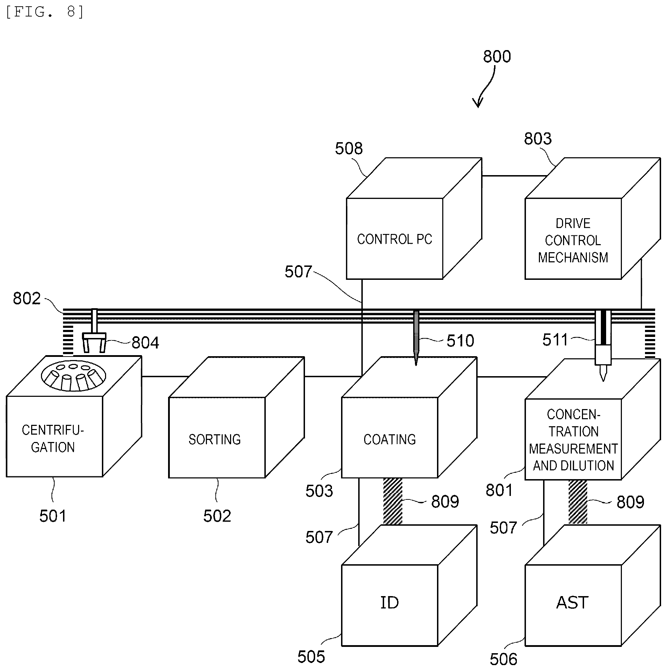

[FIG. 8] FIG. 8 is a block diagram showing a configuration of a test system according to Embodiment 2.

### Description of Embodiments

Hereinafter, embodiments according to the invention will be described with reference to the drawings.

Although the accompanying drawings show specific embodiments based on principles of the invention, these embodiments are provided for understanding the invention and are not to be used for limitedly interpreting the invention. In all the drawings for showing the embodiments, components having the same functions are denoted by the same reference numerals, and repeated description thereof is omitted.

In the following embodiments, a collection method for obtaining samples in different forms at a time from a blood sample containing a microorganism containing impurities such as blood cells, and a test method for testing a first component from the sample obtained by the collection method will be described. In the following embodiments, a container used in the collection method, a centrifuge including the container, and a test system including the centrifuge will be described.

In the following embodiments, by continuously separating a blood sample into blood cells and microorganisms, destroying the blood cells, and collecting and extracting the microorganisms, samples in different solid and liquid forms and different amounts of bacteria are obtained at a time.

The term "microorganism" to be collected refers to various types of microorganisms including bacteria, actinomyces, fungi, and the like. However, the microorganism does not contain a virus. Specifically, the microorganisms include microorganisms that are subject to detection by a sterility testing method in the pharmacopoeia, and microorganisms such as pathogenic bacteria and pathogenic fungi that are subject to tests in hospital laboratories. For example, bacteria and fungi such as escherichia (specific example: escherichia coli), staphylococcus (specific examples: staphylococcus aureus, staphylococcus epidermidis), propionibacterium (specific example, proprionobacter acnes), micrococcus, streptococcus (specific examples: streptococcus pyogenes, streptococcus pneumoniae), enterococcus (specific examples: enterococcus faecium, enterococcus faecalis), neisseria (specific examples: phosphorus bacteria, neisseria meningitidis), moraxella, shigella (specific example: shigella), salmonella (specific examples: salmonella typhi, salmonella paratyphi A, salmonella enteritidis), citrobacter, klebsiella (specific example: klebsiella pneumoniae), enterobacter, serratia (specific example: serratia marcescens), proteus, providencia, morganella, yersinia (specific example: yersinia pestis), vibrio (specific examples: vibrio cholerae, vibrio parahaemolyticus, vibrio vulnificus, vibrio mimiccus), aeromonas, pseudomonas (specific example: pseudomonas aeruginosa), acinetobacter (specific example: acinetobacter baumannii), alcaligenes, agrobacterium, flavobacterium, haemophilus (specific example: haemophilus influenzae), pasteurella, francisella, bordetella (specific example: bordetella pertussis), eikenella, brucella, streptobacillus, actinobacillus, legionella (specific example: legionella pneumophila), bacillus (specific examples: bacillus subtilis, bacillus anthracis, bacillus cereus), corynebacterium (specific example: diphtheriae), lactobacillus, Listeria, erysipelothrix, nocardia, actinomyces, clostridium (specific examples: clostridium perfringens, clostridium sporogenes), bacteroides (specific example: bacteroides fragilis), fusobacterium, mycobacterium (specific example: mycobacterium tuberculosis), campylobacter, helicobacter (specific example: helicobacter pylori), spirillum, treponema, borrelia, leptospira, mycoplasma (specific example: mycoplasma pneumoniae), aspergillus (specific examples: aspergillus niger, aspergillus brasiliensis), and yeast (specific example: Candida albicans) are included, but other microorganisms can also be collected.

The blood sample is not particularly limited as long as it is a sample containing blood cells. In particular, a biological sample derived from a living body, a sample suspected to be contaminated by a microorganism, and the like are included. For example, various samples can be used, such as blood, urine, bone marrow fluid, breast milk, amniotic fluid, biopsy tissue, cell culture fluid, and cell culture supernatant. An origin of the blood sample is not particularly limited, and it can be derived from any biological species. For example, a test sample is a blood sample derived from at least one of various types of organisms such as animals, plants, and insects. When the blood sample is a liquid sample, it can be used as it is, or diluted or concentrated with a solvent. When the blood sample is a solid sample, the blood sample may be suspended in a solvent, homogenized by a pulverizer or the like, or stirred together with a solvent to obtain a supernatant and then be used. The blood sample may be diluted with an appropriate medium or a physiological saline solution, or may be subjected to a pretreatment or the like.

The term "collecting" means separating a microorganism from a solution containing blood cells, concentrating the microorganism contained in the solution, and the like. The concentration of the microorganism that may be contained in the sample is not particularly limited.

### <Container>

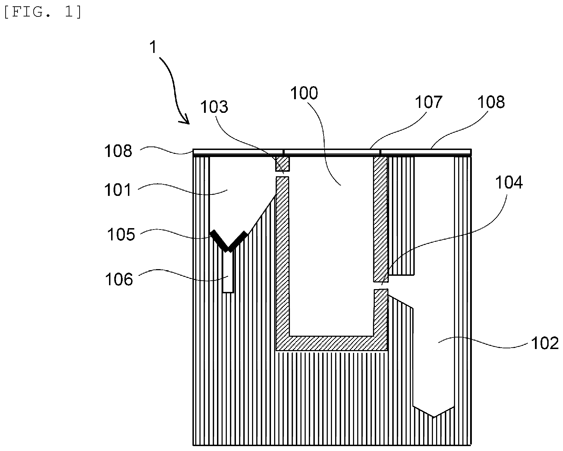

A configuration of a container 1 according to Embodiment 1 will be described with reference to FIG. 1. The container 1 includes a first storage part 100 into which a blood sample can be introduced, a second storage part 101 for collecting solid bacteria, and a third storage part 102 for collecting liquid bacteria.

A blood sample containing a blood component is introduced into the first storage part 100. Specifically, a sample positive in a blood culture test is introduced. Therefore, components of the sample to be introduced may include blood components such as red blood cells, white blood cells and platelets, a medium for bacterial growth, and bacteria.

A first flow path 103 and a second flow path 104 are provided on a side surface of the first storage part 100. The first storage part 100 is connected to the second storage part 101 via the first flow path 103. The first storage part 100 is connected to the third storage part 102 via the second flow path 104. The first flow path 103 is positioned higher than the second flow path 104. Accordingly, the blood sample at a position higher than the first flow path 103 can be moved to the second storage part 101 via the first flow path 103. The blood sample at a position higher than the second flow path 104 can be moved to the third storage part 102 through the second flow path 104.

The second storage part 101 is provided with a filtration filter 105. The second storage part 101 has a portion where the filtration filter 105 is installed. The filtration filter 105 is a filtration member that separates a solution moved from the first storage part 100 to the second storage part 101 into a microorganism and a liquid component. A waste liquid reservoir 106 is formed in the second storage part 101. The filtration filter 105 collects only solid bacterial components, and the liquid component of the solution contained in the second storage part 101 moves to the waste liquid reservoir 106.

In order to prevent contamination, the storage parts 100, 101, and 102 are preferably sealed. In the container 1 according to Embodiment 1, for example, the first storage part 100 is covered with a lid 107. Since the blood sample obtained from a blood culture bottle is introduced into the first storage part 100, the lid 107 is preferably a lid such as a rubber stopper or a seal stopper. The second storage part 101 and the third storage part 102 are covered with lids 108. The collected bacteria accumulate in the second storage part 101 and the third storage part 102. Since the collected bacteria are recovered with a needle or stick with a sharp tip, a pipette tip, or a syringe, it is preferable to use a lid made of a material that can be easily peeled off, such as a seal-like lid.

### <Microorganism Collection Method>

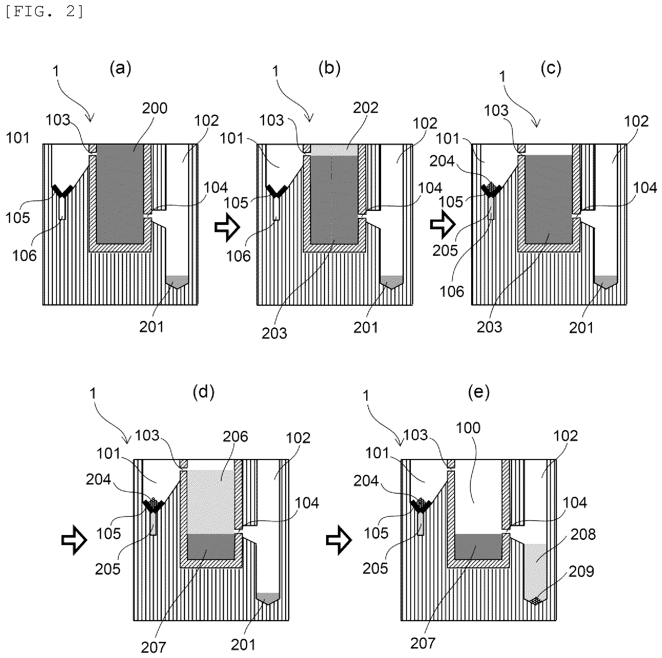

A microorganism collection method for collecting microorganisms (bacteria in Embodiment 1) from the blood sample will be described with reference to FIG. 2.

As shown in (a) of FIG. 2, a blood sample 200 is introduced into the first storage part 100 of the container 1. The introduction of the blood sample 200 into the first storage part 100 may be performed by a user or may be performed by a robot. The blood sample 200 is preferably introduced to a position sufficiently higher than the position of the first flow path 103. Next, a reagent (for example, a blood cell destruction reagent) 201 for destroying red blood cells is introduced into the third storage part 102. The introduction of the reagent 201 into the third storage part 102 may be performed by the user or may be performed by the robot. The reagent 201 is, for example, a surfactant. A concentration of the surfactant is preferably such that it does not affect cell membranes of bacteria but destroys cell membranes of red blood cells. The surfactant may be introduced after the blood sample 200 is introduced, or may be introduced into the third storage part 102 before the blood sample 200 is introduced.

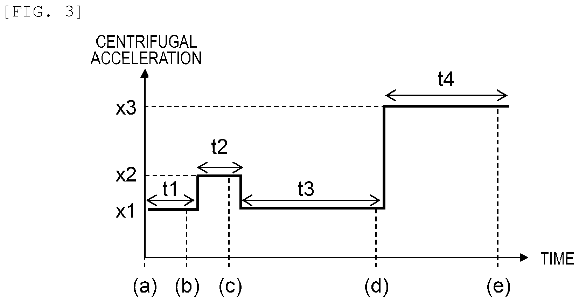

The container 1 into which the blood sample 200 is introduced is mounted on a centrifuge. Thereafter, the container 1 is centrifuged at various centrifugal accelerations according to the order of (b) to (e) of FIG. 2. The centrifuged container 1 is gradually inclined, and finally disposed so that a centrifugal force is applied toward a bottom surface of the container 1. At this time, directions of the first flow path 103 and the second flow path 104 of the container 1 are different from a direction in which the centrifugal force is applied. Details of the centrifugal acceleration and a centrifugal time applied to the container 1 will be described later with reference to FIG. 3.

First, the blood sample 200 stored in the container 1 is centrifuged at a low acceleration. Then, as shown in (b) of FIG. 2, the blood sample 200 is separated into a bacterial solution (first solution) 202 containing bacteria (first component) and a solution (second solution) 203 containing heavy particles such as red blood cells or white blood cells (second component). In addition to the bacteria, the bacterial solution 202 contains plasma, a medium, and the like, and may contain red blood cells and white blood cells that are not completely separated in some cases.

Next, the blood sample 200 is centrifuged at an acceleration slightly higher than that of the centrifugation at an initial low acceleration. Then, as shown in (c) of FIG. 2, the centrifugal force is more than a pressure of the liquid in the first flow path 103, and the bacterial solution 202 moves to the second storage part 101 via the first flow path 103. Thereafter, the bacterial solution 202 is in contact with the filtration filter 105, and bacteria (first sample) 204 as a solid component are collected on the filtration filter 105. The filtration filter 105 may be any filter, and for example, a membrane filter having a diameter of 0.1 um or less is preferable, and a hydrophilic filter is more preferable, and a material such as PVDF is preferable. The diameter of the filtration filter 105 may be any value as long as the filter can collect the bacteria 204, and any diameter of several um or less is suitable for use. A liquid component 205 that passes through the filtration filter 105 contains plasma, a medium, proteins that are sufficiently smaller than bacteria, and the like, and moves to the waste liquid reservoir 106. In this manner, the bacteria 204 contained in the bacterial solution 202 stored in the second storage part 101 are collected on the filtration filter 105.

Next, the solution 203 is centrifuged at the same low acceleration as at the beginning. Then, as shown in (d) of FIG. 2, the solution 203 in the first storage part 100 is separated into a bacterial solution (third solution) 206 containing bacteria and a blood cell component (fourth solution) 207 containing heavy particles such as red blood cells and white blood cells.

Finally, the solution 203 is centrifuged at a highest acceleration. Then, as shown in (e) of FIG. 2, the centrifugal force is more than a pressure of the liquid in the second flow path 104, and the bacterial solution 206 moves to the third storage part 102 through the second flow path 104. The bacterial solution 206 moved to the third storage part 102 is mixed with the reagent 201, and a minute amount of blood cell components remaining without being separated by centrifugation is destroyed. Thereafter, by subsequent centrifugation, the bacterial solution 206 moved to the third storage part 102 is separated into a liquid component 208 and bacteria (second sample) 209. The bacteria 209 accumulate on a bottom surface of the third storage part 102 and can be collected.

As a method for improving accuracy of analysis, the following method can be considered. For example, impurities contained in the blood sample 200 may be mixed into the bacteria 204 collected in the second storage part 101 and the bacteria 209 collected in the third storage part 102. In this case, for example, the liquid component 208 is first removed from the state of (e) of FIG. 2. Thereafter, an appropriate amount of a surfactant or a physiological saline solution is added dropwise to the bacteria 204 and 209, and the bacteria 204 and 209 are centrifuged and washed again at a high acceleration, whereby a sample of bacteria with higher purity can be obtained without complicated operation.

Next, a relationship between the centrifugal acceleration applied to the container 1 and the centrifugal time will be described with reference to FIG. 3. States of the container 1 at times (a) to (e) in FIG. 3 correspond to the states (a) to (e) in FIG. 2, respectively.

First, the blood sample 200 is centrifuged at a centrifugal acceleration (first centrifugal force) x1 for a time t1. At this time, the centrifugal acceleration x1 needs to be set so that the pressure in the first flow path 103 is greater than the centrifugal force so that the blood sample 200 in the first storage part 100 does not move to the second storage part 101 via the first flow path 103. In addition, since it is necessary to perform the centrifugation until an interface position between the bacterial solution 202 and the solution 203 is lower than the height of the first flow path 103, the centrifugal time t1 is determined based on a sedimentation speed of blood cells and the height of the first flow path 103. Specifically, although it is affected by the diameter of the first flow path 103, the material of the container 1, and physical properties (for example, viscosity) of the blood sample 200, the centrifugal acceleration x1 is preferably 20 to 100 G, for example.

Next, the blood sample 200 is centrifuged at a centrifugal acceleration (second centrifugal force) x2 for a time t2. The centrifugal acceleration x2 needs to be greater than the centrifugal acceleration x1. The centrifugal acceleration x2 is set so that the centrifugal force is greater than the pressure in the first flow path 103. Then, the first flow path 103 can no longer hold the liquid, and the bacterial solution 202 moves to the second storage part 101. The pressure in the first flow path 103 is determined by a surface tension as well as the diameter and a length of the first flow path 103. In the state of (b) of FIG. 2, the first flow path 103 and the second flow path 104 are in contact with different liquids. The first flow path 103 is in contact with the bacterial solution 202 containing almost no blood cells, and the second flow path 104 is in contact with the solution 203 containing blood cells. In this way, since the liquid in contact with the first flow path 103 and the liquid in contact with the second flow path 104 have different surface tensions, the solution 203 does not move from the second flow path 104, which is in contact with the liquid having a high surface tension.

Next, the solution 203 is centrifuged at the centrifugal acceleration (third centrifugal force) x1 for a time t3. At this time, the centrifugal acceleration x1 needs to be set so that the pressure in the second flow path 104 is greater than the centrifugal force so that the liquid does not move from the first storage part 100 to the third storage part 102 through the second flow path 104. Since it is necessary to perform the centrifugation until an interface position between the bacterial solution 206 and the blood cell component 207 is lower than the height of second flow path 104, the centrifugal time t3 is determined based on the sedimentation speed of blood cells and the height of second flow path 104.

Finally, the solution 203 is centrifuged at a centrifugal acceleration (fourth centrifugal force) x3 for a time t4. In order to rapidly move the bacterial solution 206 from the second flow path 104 to the third storage part 102, the centrifugal acceleration x3 is preferably a sufficiently high value. However, in consideration of the possibility of affecting growth of bacteria in the bacterial solution 206, the centrifugal acceleration is preferably as high as possible in a range of 10⁴ G or less. When the centrifugation is performed at the centrifugal acceleration x3, the bacteria 204 and 209 are collected in the second storage part 101 and the third storage part 102, respectively. The solid bacteria 204 are collected on the filtration filter 105 provided in the second storage part 101, and the highly concentrated bacteria 209 are collected on the bottom surface of the third storage part 102.

### <Height of Flow Path Formed in Container 1>

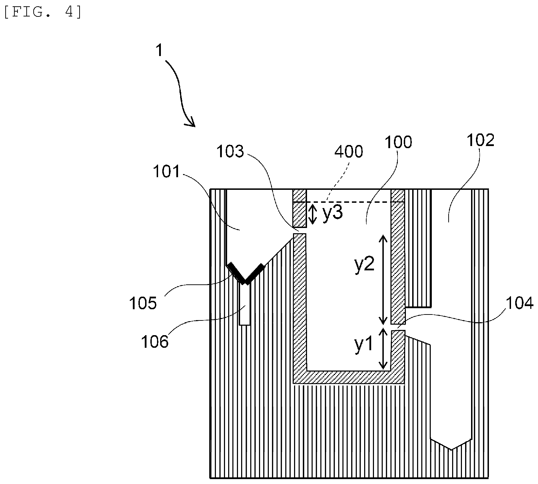

The heights of the first flow path 103 and the second flow path 104 formed in the container 1 will be described with reference to FIG. 4. The first flow path 103 and the second flow path 104 have different heights. The reason for this is to automatically collect bacteria in different forms used in different tests. Two types of tests are considered, that is, an identification test using MALDI and a sensitivity test. For example, in the case of the identification test using MALDI, bacteria of 10⁵ to 10⁶ CFU or more are required. In the sensitivity test, a bacterial solution of 10⁵ to 10⁶ CFU/mL is required, and for example, several hundred µL of the bacterial solution whose concentration is adjusted to 1.5 × 10⁸ CFU/mL (equivalent to 0.5 McFarland) is prepared by McFarland turbidimetry, and then diluted 100 to several hundred times and used for testing.

In order to consider the heights of the first flow path 103 and the second flow path 104, a positive blood culture sample is considered. A liquid amount of the positive blood culture sample introduced into the first storage part 100 was 10 mL. The concentration of the bacteria contained in the positive blood culture sample varies depending on a time until the blood culture is positive, a time elapsed since the blood culture is positive, and bacterial species and strain, and is generally in a range of 10⁷ to 10⁹ CFU/mL. Therefore, a case is considered in which a bacterial concentration is 10⁷ CFU/mL, which is expected to be the most difficult pretreatment.

As a premise, the heights of the first flow path 103 and the second flow path 104 are as shown in FIG. 4. y1 is a height from the bottom surface of the first storage part 100 to the second flow path 104. y2 is a height from the second flow path 104 to the first flow path 103. y3 is a height from the first flow path 103 to a scale line 400 indicating a specified value. y1 + y2 + y3 is a height of the blood sample 200 when the entire expected amount of the blood sample 200, that is, 10 mL, is added. The container 1 is provided with the visually recognizable scale line 400 at a position of the height of y1 + y2 + y3. It is necessary to introduce at least the blood sample 200 of the specified value or more into the first storage part 100.

First, the height y1 from the bottom surface of the first storage part 100 to the second flow path 104 is determined as follows. Since the centrifuged red blood cells are deposited in the first storage part 100, a volume of the first storage part 100 from the bottom surface of the first storage part 100 to the height y1 needs to be equal to or greater than a volume of the red blood cells in the blood sample 200. Here, a hematocrit value, which indicates a ratio of red blood cells in blood, is in a range from 30 to 55%, depending on gender and health condition. The blood sample 200 to be introduced into the first storage part 100 contains, in addition to blood of a patient, about 2/3 of the total volume of a medium component introduced in advance. Therefore, it can be said that the ratio of red blood cells in the positive blood culture sample is about 1/6 at the maximum. Therefore, in order to more efficiently pretreat the blood sample, the height of y1 is preferably 16.7% or more with respect to a specified height of an input amount of the blood sample 200, and is set to, for example, 20%.

Next, the height of y3 is determined as follows. When the bacterial concentration is 10⁷ CFU/mL, bacteria of 10⁷ CFU can be extracted if the sample is 1 mL. This is more than 10 times the amounts of bacteria required for MALDI, and it is considered that sufficient amounts of bacteria can be extracted. Therefore, the height of y3 is preferably about 10% of the specified height of the input amount of the blood sample 200.

From the above calculation, the height of y2 is automatically determined. That is, the height of y2 is about 70% of the specified height of the input amount of the blood sample 200. In this case, the amount of bacteria extracted near the bottom surface of the third storage part 102 is 10⁷ CFU/mL × 7 mL = 7 × 10⁷ CFU. In actual operations, such a small amount of highly concentrated bacterial solution is diluted to several hundred µL, for example, 200 µL, and the concentration is adjusted to 1.5 × 10⁸ CFU/mL (equivalent to 0.5 McFarland). According to the calculation, it is possible to extract 3.5 × 10⁸ CFU/mL of bacterial solution while 1.5 × 10⁸ CFU/mL of bacterial solution is required, and thus it is possible to extract a sufficient amount even in consideration of loss of bacteria during separation.

An example of the calculation is an assumption that the positive blood culture sample has a low bacterial concentration. In practice, the bacterial concentration in the positive blood culture sample is often 10⁸ CFU/mL or more, and the bacteria can be extracted with sufficient margin.

In addition to the heights of the first flow path 103 and the second flow path 104, there are the following factors characterizing the container 1.

As the container 1, a container into which the blood sample 200 can be introduced and which can be subjected to the centrifugation can be used. For example, the volume of the first storage part 100 may be 1 to 20 mL, and preferably 8 to 15 mL. A sum of the volume of the second storage part 101 and the volume of the third storage part 102 may be equal to or greater than the volume of the first storage part 100. Since the amount of bacteria collected in the third storage part 102 is greater than that in the second storage part 101, it is preferable that the volume of the third storage part 102 is large. The material of the container 1 is not particularly limited as long as the material is suitable for operations such as the centrifugation. For example, the container 1 is preferably made of a hydrophobic material. This is because when affinity between the container 1 and the blood sample 200 is good, the blood sample 200 may be moved from the first flow path 103 or the second flow path 104 before the centrifugation due to the surface tension. For example, the container 1 is preferably made of a material such as an acrylic resin, an ABS resin, a polypropylene, a polystyrene, or a polyethylene, and is made using a 3D printer or injection molding. Alternatively, the container 1 may be produced by cutting using aluminum or stainless steel. In order to make the container 1 hydrophobic, the container 1 may be subjected to a chemical treatment for surface modification. The container 1 may be non-transparent, and in the case of being transparent, it is easy to visually check an internal state, or to perform optical measurement and imaging using a device. The container 1 is preferably transparent in order to facilitate detection of excess or deficiency in the liquid amount, clogging of the sample, and detection of a foreign matter in the sample. From the viewpoint of contamination, the container 1 is preferably disposable, whereas there is no problem if the container 1 is used repeatedly, for example, after being washed and sterilized.

An outer shape of the container 1 may be any shape as long as it can be centrifuged. A bottom surface portion of the second storage part 101 on which the filtration filter 105 is installed and a bottom surface portion of the third storage part 102 in which the reagent is stored preferably have a mortar shape in order to efficiently collect the bacteria 204 and 209.

The first flow path 103 and the second flow path 104 may have any shape. Each of the first flow path 103 and the second flow path 104 has, for example, a cylindrical shape having a circular cross-sectional shape or a rectangular parallelepiped shape having a long rectangular cross-sectional shape. The diameters of the first flow path 103 and the second flow path 104 are considered as follows. Blood contains red blood cells, white blood cells, and platelets, and the largest of these is the white blood cell, which has a diameter of 6 to 30 um. When the diameters of the first flow path 103 and the second flow path 104 are smaller than the above values, the blood cells may clog the first flow path 103 and the second flow path 104 rather than an effect of a capillary valve, and the liquid may not be released. Therefore, although depending on surface affinity of the blood sample 200 and the container 1, a range of actual optimum values of the diameters of the first flow path 103 and the second flow path 104 is 10 to 100 um in continuously performing the separation of the blood cells and the liquid components and a movement thereof from the first flow path 103 and the second flow path 104. The diameter of the first flow path 103 and the diameter of the second flow path 104 may be different, but are preferably the same.

The reagent 201 introduced into the third storage part 102 is not particularly limited as long as it is a reagent capable of destroying the blood cells without affecting the growth of microorganisms. For example, the reagent 201 preferably contains at least one surfactant. Examples of the surfactant include, but are not limited to, an anionic surfactant that has a hydrophilic part and a hydrophobic part, the hydrophobic part being a chain hydrocarbon, a surfactant that has a hydrophilic part and a hydrophobic part, the hydrophobic part having a cyclic hydrocarbon, and a combination of both. Specifically, the former includes sodium dodecyl sulfate, lithium dodecyl sulfate, and sodium N-lauroylsarcosine, and the latter includes saponin, sodium cholate, sodium deoxycholate, 3-[(3-cholamidopropyl)dimethylammonio]-1-propanesulfonate, and 3-[(3-cholamidopropyl)dimethylammonio]-2-hydroxy-1-propanesulfonate.

### <Test System>

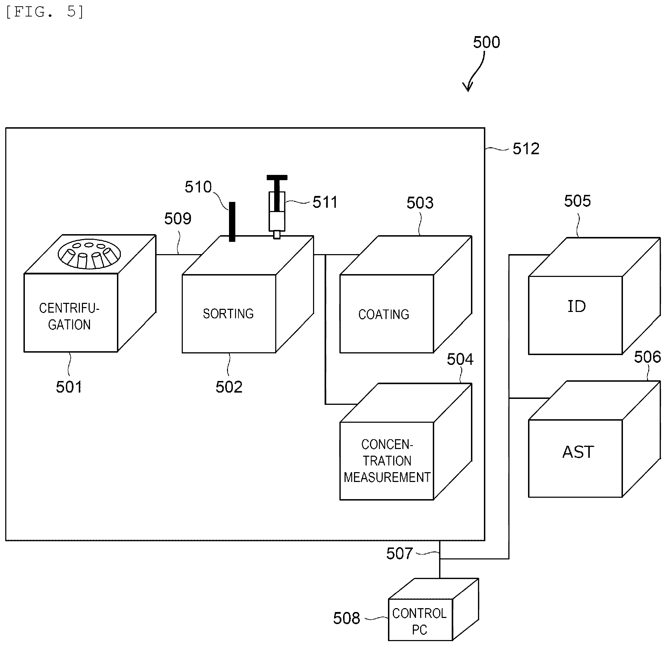

A test system 500 that automatically performs a bacteria test will be described with reference to FIG. 5. The test system 500 includes a centrifuge 501, a sorting device 502, a coating device 503, a concentration measurement device 504, an identification test device 505, a sensitivity test device 506, and a control PC 508. Each device is connected by a signal line 507, and the control PC 508 controls an operation of each device.

The centrifuge 501 is capable of mounting one or more containers 1 described above, and centrifuges the blood sample 200 introduced into the first storage part 100 of the mounted container 1 to separate the bacteria 204 used in the identification test device 505 and the bacteria 209 used in the sensitivity test device 506. The centrifuge 501 centrifuges the blood sample 200 with at least three accelerations set for centrifugation of the blood sample 200, a movement of the bacterial solution 202 to the second storage part 101, and a movement of the bacterial solution 206 to the third storage part 102. The container 1 is transported to the sorting device 502 by a transport mechanism 509. The user may transport the container 1 from the centrifuge 501 to the sorting device 502.

The sorting device 502 acquires the bacteria 204 collected in the second storage part 101 of the container 1 using, for example, a specimen collecting rod 510 with a sharp tip, and acquires the bacteria 209 collected in the third storage part 102 of the container 1 using a syringe 511. The obtained bacteria 204 and 209 are sent to different devices.

The coating device 503 coats solid bacteria and a matrix reagent onto a MALDI target plate used in mass spectrometry for the identification test.

The concentration measurement device 504 measures a concentration of the bacterial solution, for example, by measuring McFarland turbidity.

A pretreatment device 512 includes the centrifuge 501, the sorting device 502, the coating device 503, and the concentration measurement device 504. However, the coating device 503 may not necessarily exist as a necessary component. For example, the sorting device 502 may return the specimen collecting rod 510 that acquires the bacteria 204 to the user, and the user may apply the specimen collecting rod 510 to a target plate by himself/herself.

A concentration value measured by the pretreatment device 512 may be displayed on a display attached to the control PC 508 to notify the user. When the concentration value is equal to a target value, for example, reliability of the test can be maintained by notifying the user and adjusting the concentration value to the target value by the user. When the concentration is equal to or lower than the target value, an error flag may be displayed, and the pretreatment may be performed again, or a method may be adopted in which the bacterial solution is additionally cultured and then used for testing.

In addition, as an additional function, the sorting device 502 may have a function of acquiring appearance information of the container 1 using, for example, concentration measurement or image measurement and confirming whether the processing of a previous stage is normally executed. For example, when the processing of the previous stage is normally performed, a liquid surface height of the blood sample 200 in the first storage part 100 is about the same as the height of the second flow path 104 as shown in (e) of FIG. 2. However, when the processing of the previous stage is stopped in the middle, the liquid surface height of the blood sample 200 in the first storage part 100 is sufficiently higher than the height of the second flow path 104. Based on such information, the sorting device 502 can verify validity of whether the processing of the previous stage is normally completed.

A sample processed by the pretreatment device 512 is returned to the user once, subjected to an additional processing step or the like as necessary, and then tested by the identification test device 505 and the sensitivity test device 506. The identification test device 505 determines a type of bacteria using the sample obtained from the bacteria 204. The sensitivity test device 506 determines a degree of growth of bacteria using the sample obtained from the bacteria 209. In the case of the identification test, bacterial species is identified by a device, possibly after the user applies the bacteria and matrix reagent to the target plate by himself/herself. In the case of the sensitivity test, for example, adjustment of the bacterial solution to a target concentration and dispensing to a 96-well plate or the like are performed, and the sensitivity test device 506 performs determination of growth and MIC. A progress status and a determination result of the identification of bacterial species and a determination of the sensitivity test are displayed on a display attached to the control PC 508, and the result is notified to the user. In addition, it is preferable that the control PC 508 inputs parameters of each device such as an acceleration and a time of the centrifugation in the centrifuge 501 and a target value of the bacteria concentration in the concentration measurement device 504 according to the blood sample 200, and can change a processing content according to the value.

### <Centrifuge>

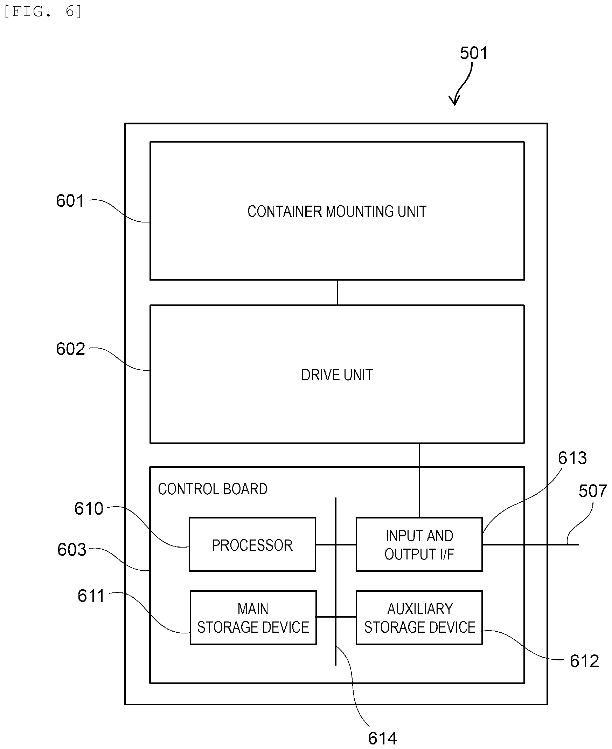

The centrifuge 501 will be described in detail with reference to FIG. 6. As shown in FIG. 6, the centrifuge 501 includes a container mounting unit 601, a drive unit 602, and a control board 603. One or a plurality of containers 1 are mounted on the container mounting unit 601. The drive unit 602 is an actuator that applies a centrifugal force to the blood sample 200 in the container 1 mounted on the container mounting unit 601. The control board 603 is a control unit that controls an operation of the drive unit 602. The control board 603 includes a processor 610, a main storage device 611, an auxiliary storage device 612, an input and output I/F 613, and a bus 614 that communicably connects each unit of the control board 603. The input and output I/F 613 is communicably connected to the control PC 508 via the signal line 507, and receives an instruction from the control PC 508. The processor 610 generates a drive signal for driving the drive unit 602 according to an instruction from the control PC 508. The input and output I/F 613 is communicably connected to the drive unit 602, and outputs a drive signal generated by the processor 610 to the drive unit 602. The drive unit 602 centrifuges the container 1 at various centrifugal accelerations according to the drive signal.

### <Bacterial Test Method>

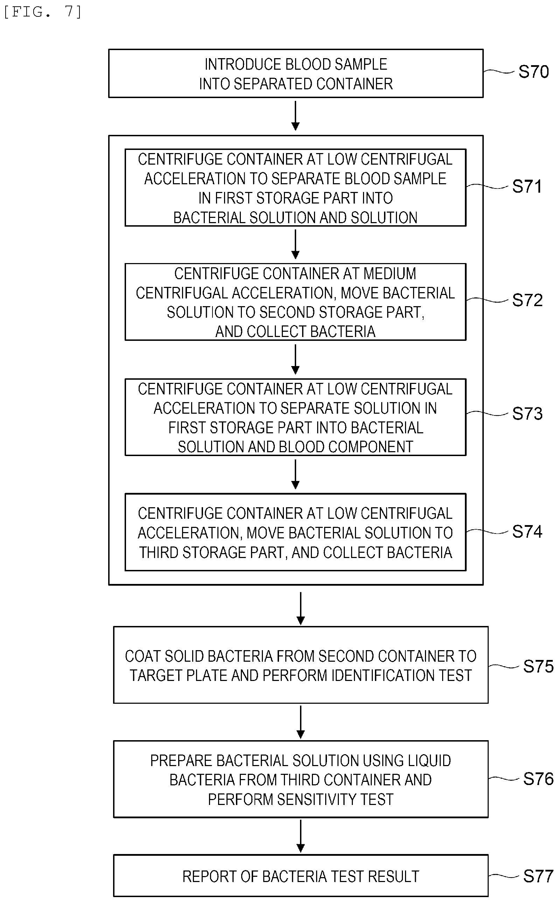

A bacterial test method according to Embodiment 1 will be described with reference to FIG. 7. Each process of S71 to S74 in FIG. 7 is executed in the centrifuge 501 according to a command from the control board 603, which is a computer system of the centrifuge 501.

First, in step S70, the blood sample 200 is introduced into the first storage part 100 of the container 1. The process of introducing the blood sample 200 into the container 1 may be performed by the user or may be performed by the robot. The reagent 201 such as a surfactant that destroys blood cells may be introduced into the third storage part 102 in step S70, or may be introduced into the third storage part 102 before step S70.

Although steps S71 to S74 are individually shown in FIG. 7, a series of processes of steps S71 to S74 is automatically and continuously performed by the centrifuge 501. In step S71, the container 1 storing the blood sample 200 is centrifuged at a low acceleration to separate the blood sample 200 into the bacterial solution 202 and the solution 203. In step S72, the container 1 storing the blood sample 200 is centrifuged at a medium acceleration, and the bacterial solution 202 is moved from the first storage part 100 to the second storage part 101 through the first flow path 103. Then, the liquid component 205 of the bacterial solution 202 moved to the second storage part 101 is removed by the filtration filter 105, and the bacteria 204 are collected. In step S73, the container 1 storing the solution 203 is centrifuged at a low acceleration, and the solution 203 is separated into the bacterial solution 206 and the blood cell component 207. Then, in step S74, the container 1 storing the solution 203 is centrifuged at a high acceleration, and the bacterial solution 206 is moved from the first storage part 100 to the third storage part 102 through the second flow path 104. In the third storage part 102, the reagent 201 destroys the red blood cells remaining in the bacterial solution 206 and elutes them into the liquid. As a result, only the bacterial component accumulates on the bottom surface of the third storage part 102.

In step S75, the identification test device 505 performs an identification test using the bacteria 204 collected in the second storage part 101. For example, for the identification test using MALDI, the identification test may be performed by obtaining a mass of the solid bacteria 204 collected on the filtration filter 105 of the second storage part 101, applying the mass to the target plate, dropping an appropriate matrix reagent, and drying. In order to improve detection sensitivity of the identification test, there is no problem also in performing a protein extraction process using ethanol, formic acid, acetonitrile, and the like, as in the usual MALDI method.

In step S76, the sensitivity test device 506 acquires the highly concentrated bacterial solution accumulated on the bottom surface of the third storage part 102, and performs the sensitivity test. Specifically, the highly concentrated bacteria 209 accumulated on the bottom surface are acquired with a pipette tip or the like, or the highly concentrated bacteria 209 accumulated on the bottom surface after removing the supernatant are acquired. Then, the concentration of the bacterial solution is adjusted by, for example, McFarland turbidimetry, and the sensitivity test is performed using the bacterial solution.

In step S77, a bacteria test result is reported. Specifically, the identification test performed in S75 is used to report information on bacterial species and a determination score. In addition, through the sensitivity test performed in S76, information such as a drug name, minimum inhibitory concentration (MIC) of a drug, and sensitivity/resistance is reported to a test technician and a doctor.

### <Effects of Embodiment 1>

In Embodiment 1, the container 1 including the first storage part 100 having the first flow path 103 and the second flow path 104 of different heights on a side surface, the second storage part 101 connected to the first storage part 100 via the first flow path 103, and the third storage part 102 connected to the first storage part 100 via the second flow path 104 is centrifuged at various centrifugal accelerations. Accordingly, it is possible to separate blood cells and bacteria from the blood sample 200 introduced into the first storage part 100. Further, the blood sample 200 stored in the first storage part 100 is centrifuged, so that the bacterial solution 202 can be moved to second storage part 101 via the first flow path 103, and the bacterial solution 206 can be moved to the third storage part 102 via the second flow path 104. Accordingly, it is possible to collect the solid bacteria 204 and the liquid bacteria 209 having different forms in the second storage part 101 and the third storage part 102, respectively. As described above, in Embodiment 1, bacterial samples in different forms to be used in the identification test and the sensitivity test can be acquired at a time by a simple and stable method.

As described above, after the blood sample is introduced, if a series of centrifugation operations are performed once at a predetermined centrifugal acceleration, the bacterial samples in different forms are extracted. A solid bacterial sample with a small amount of liquid component is suitable for use in mass spectrometry using MALDI, and a liquid bacterial sample is suitable for use in a sensitivity test. Since an amount of bacteria to be extracted can be controlled by the heights of the first flow path 103 and the second flow path 104, the user can automatically acquire a prescribed amount or more of the bacterial samples suitable for each test without complicated operations.

Usually, sample preparation equivalent to acquiring bacteria from a colony after separation culture and preparing a bacterial solution, which requires about one day and a night, can be achieved with about ten minutes of one pretreatment. In the known method, repeated centrifugation is required for a plurality of times of separation, destruction processes, and washing steps, and different samples for the identification test and the sensitivity test cannot be obtained by one pretreatment, whereas in the invention, a series of steps are performed continuously, thereby enabling a rapid test.

In Embodiment 1, the solid bacteria 204 can be collected by the filtration filter 105 of the second storage part 101. Accordingly, solid bacteria necessary for the identification test can be acquired.

In addition, in Embodiment 1, the reagent 201 introduced into the third storage part 102 does not affect the cell membranes of the bacteria in the bacterial solution 206, but can destroy the cell membranes of the red blood cells. Accordingly, it is possible to destroy a trace amount of blood cell components in the bacterial solution 206 remaining without being separated by the centrifugation.

In Embodiment 1, the heights of the first flow path 103 and the second flow path 104 are equal to or greater than 1/6 of the height from the bottom surface of the first storage part 100 to a specified value, and a difference (y2) between the height of the first flow path 103 and the height of the second flow path 104 is equal to or greater than 1/2 of the height from the bottom surface of the first storage part 100 to the specified value. In addition to blood of a patient, it is possible to acquire the bacterial samples in an amount necessary for the identification test and the sensitivity test from the blood sample 200, which contains about 2/3 of a total volume of medium components.

In addition, in Embodiment 1, the solution 203 is centrifuged at the centrifugal acceleration x3 that is sufficiently greater than the centrifugal acceleration x2, and thus the bacterial solution 206 can be rapidly moved from the second flow path 104 to the third storage part 102.

In Embodiment 1, the type of bacteria can be determined using the bacteria 204, and a degree of growth of the bacteria can be determined using the bacteria 209.

In addition, in Embodiment 1, since the bottom surface of each of the second storage part 101 and the third storage part 102 of the container 1 has a mortar shape, the collected bacteria 204 and 209 can be efficiently acquired with a sharp needle or stick, a pipette tip, or a syringe.

### (Embodiment 2)

In Embodiment 1, an example in which the user manually acquires the bacteria 204 collected in the second storage part 101 and the bacteria 209 collected in the third storage part 102 using a needle or a pipette tip is described. In Embodiment 2, the bacteria 204 collected in the second storage part 101 are automatically sorted and coated, and the bacteria 209 collected in the third storage part 102 are automatically sorted, diluted, and measured in concentration.

Similarly to Embodiment 1, a test system 800 according to Embodiment 2 includes the centrifuge 501, the sorting device 502, the coating device 503, the identification test device 505, the sensitivity test device 506, and the control PC 508. Further, the test system 800 according to Embodiment 2 includes a concentration measurement and dilution device 801, a drive mechanism 802, a drive control mechanism 803, a gripping mechanism 804, and a transport device 809.

The drive mechanism 802 is controlled by the drive control mechanism 803. The drive mechanism 802 moves the gripping mechanism 804 from the centrifuge 501 to the sorting device 502. The gripping mechanism 804 is implemented to be able to grip the container 1, and grips the container 1 centrifuged by the centrifuge 501. The drive mechanism 802 moves the specimen collecting rod 510 from the sorting device 502 to the coating device 503. Further, the drive mechanism 802 moves the syringe 511 from the sorting device 502 to the concentration measurement and dilution device 801. A sample produced by the coating device 503 is transported to the identification test device 505 by the transport device 809. The sample whose concentration is measured and diluted by the concentration measurement and dilution device 801 is transported to the sensitivity test device 506 by the transport device 809.

### <Effects of Embodiment 2>

The test system 800 according to Embodiment 2 can automatically perform the process from the centrifugation in the centrifuge 501 to the test in the identification test device 505 and the test in the sensitivity test device 506.

The invention is not limited to the above-described embodiments, and includes various modifications. The above-described embodiments have been described in detail to facilitate understanding of the invention, and the invention is not necessarily limited to those including all the configurations described above. A part of a configuration in one embodiment can be replaced with a configuration in another embodiment, and a configuration in one embodiment can also be added to a configuration in another embodiment. A part of a configuration in each embodiment may also be added to, deleted from, or replaced with another configuration.

### Reference Signs List

1: container
100: first storage part
101: second storage part
102: third storage part
103: first flow path
104: second flow path
105: filtration filter
106: waste liquid reservoir
107: lid
108: lid
200: blood sample
201: reagent
202: bacterial solution
203: solution
204: bacteria
205: liquid component
206: bacterial solution
207: blood cell component
208: liquid component
209: bacteria
400: scale line
500: test system
501: centrifuge
502: sorting device
503: coating device
504: concentration measurement device
505: identification test device
506: sensitivity test device
507: signal line
508: control PC
509: transport mechanism
510: specimen collecting rod
511: syringe
512: pretreatment device
601: container mounting unit
602: drive unit
603: control board
610: processor
611: main storage device
612: auxiliary storage device
613: input and output I/F
614: bus
800: test system
801: concentration measurement and dilution device
802: drive mechanism
803: drive control mechanism
804: gripping mechanism
809: transport device

## Claims

1. A collection method comprising:
introducing a sample into a first storage part of a container including the first storage part having a first flow path and a second flow path of different heights on a side surface, a second storage part connected to the first storage part via the first flow path, and a third storage part connected to the first storage part via the second flow path;
centrifuging the sample introduced into the first storage part with a first centrifugal force to separate the sample into a first solution containing a first component and a second solution containing a second component;
centrifuging the sample stored in the first storage part with a second centrifugal force greater than the first centrifugal force and moving the first solution to the second storage part via the first flow path;
centrifuging the second solution stored in the first storage part with a third centrifugal force to separate the second solution into a third solution containing the first component and a fourth solution containing the second component; and
centrifuging the second solution stored in the first storage part with a fourth centrifugal force greater than the third centrifugal force and moving the third solution to the third storage part via the second flow path.

2. The collection method according to claim 1, further comprising:
separating the first solution into the first component and a liquid component by a filtration member provided in the second storage part.

3. The collection method according to claim 1, further comprising:
destroying a cell membrane of the second component contained in the third solution by a reagent stored in the third storage part.

4. The collection method according to claim 1, wherein
the first flow path is provided at a position higher than that of the second flow path,
introducing the sample into the first storage part includes introducing the sample into the first storage part to a level equal to or higher than a specified value higher than that of the first flow path,
moving the first solution to the second storage part includes moving the first solution, which is in an amount determined based on a difference between a height of the sample introduced into the first storage part and a height of the first flow path, to the second storage part via the first flow path, and
moving the third solution to the third storage part includes moving the third solution, which is in an amount determined based on a difference between the height of the first flow path and a height of the second flow path, to the third storage part via the second flow path.

5. The collection method according to claim 4, wherein
the height of the first flow path and the height of the second flow path are 1/6 or more of a height from a bottom surface of the first storage part to the specified value, and
the difference between the height of the first flow path and the height of the second flow path is 1/2 or more of the height from the bottom surface of the first storage part to the specified value.

6. The collection method according to claim 1, wherein
the fourth centrifugal force is greater than the second centrifugal force.

7. A test method comprising:
acquiring a first sample from the first solution moved to the second storage part by the collection method according to claim 1;
acquiring a second sample from the third solution moved to the third storage part by the collection method according to claim 1;
determining a type of the first component by using the first sample; and
determining a degree of growth of the first component by using the second sample.

8. A container to be mounted on a centrifuge, the container comprising:
a first storage part configured to allow a sample to be introduced in and having a first flow path and a second flow path of different heights on a side surface;
a second storage part connected to the first storage part via the first flow path; and
a third storage part connected to the first storage part via the second flow path.

9. The container according to claim 8, wherein
the second storage part includes a portion in which a filtration member that separates a solution moved from the first storage part to the second storage part into a first component and a liquid component is installed.

10. The container according to claim 8, wherein
the third storage part includes a portion that stores a reagent that destroys a cell membrane of a second component contained in a solution moved from the first storage part to the third storage part.

11. A centrifuge comprising:
the container according to claim 8;
a drive unit configured to centrifuge the sample introduced into the first storage part of the container; and
a control unit configured to control a centrifugal force applied to the sample by the drive unit, wherein
the control unit
controls the drive unit to centrifuge the sample introduced into the first storage part with a first centrifugal force to separate the sample into a first solution containing a first component and a second solution containing a second component,
controls the drive unit to centrifuge the sample stored in the first storage part with a second centrifugal force greater than the first centrifugal force, and moves the first solution to the second storage part via the first flow path,
controls the drive unit to centrifuge the second solution stored in the first storage part with a third centrifugal force to separate the second solution into a third solution containing the first component and a fourth solution containing the second component, and
controls the drive unit to centrifuge the second solution stored in the first storage part with a fourth centrifugal force greater than the third centrifugal force, and moves the third solution to the third storage part via the second flow path.

12. The centrifuge according to claim 11, wherein
the control unit controls the drive unit such that the fourth centrifugal force is greater than the second centrifugal force.

13. A test system comprising:
the centrifuge according to claim 11;
a sorting device configured to acquire a first sample from the second solution stored in the second storage part of the container and acquire a second sample from the third solution stored in the third storage part;
an identification test device configured to determine a type of the first component using the first sample acquired by the sorting device; and
a sensitivity test device configured to determine a degree of growth of the first component using the second sample acquired by the sorting device.
